# EUROPEAN PATENT APPLICATION

(11) **EP 4 374 819 A1**
(43) Date of publication of application: **29.05.2024**
(21) Application number: 22846064.8
(22) Date of filing: 27.06.2022
(51) Int. Cl.: A61C 13/00, G06F 30/20, G06T 17/00, G16H 50/50

(54) **IMAGE PROCESSING APPARATUS AND IMAGE PROCESSING METHOD**

(30) Priority: 21.07.2021 KR 20210095700; 23.06.2022 KR 20220076925
(71) Applicant: Medit Corp., Seoul 07207 (KR)
(72) Inventor: ROSLYAKOVA, Maria, Seoul 07207 (KR); KOO, Minkyo, Seoul 07207 (KR)
(74) Representative: Taor, Simon Edward William
(86) International application number: PCT/KR2022/009096
(87) International publication number: WO 2023/003192

(57) **Abstract**

Disclosed embodiments relate to an image processing method and an image processing apparatus. An image processing method according to an embodiment may include obtaining an oral image including data about at least one tooth and data about a restoration, obtaining, based on the oral image, an intersection area where a first tooth of the at least one tooth and the restoration overlap each other, and obtaining a final image of the restoration by cutting data corresponding to the intersection area from the data about the restoration.

## Description

### Technical Field

Disclosed embodiments relate to an image processing apparatus and an image processing method, and more particularly, to an image processing apparatus and an image processing method for processing an image of a dental restoration.

### Background Art

When a dental restoration is designed using computer-aided design/computer-aided manufacturing (CAD/CAM), it is necessary to cut an intersection occurring between a restoration of a tooth needing to be restored (hereinafter, referred to as a target tooth) and an opposing tooth or between the restoration and an adjacent tooth.

When a restoration is actually applied to a patient's oral cavity, if an intersection occurs between the restoration and an opposing tooth, the patient may feel uncomfortable when chewing food, and if an intersection occurs between the restoration and an adjacent tooth, the restoration may not be properly attached or worn. Accordingly, it is necessary to design the restoration so that there is no intersection between the restoration and the opposing tooth or the adjacent tooth.

When a restoration is designed using existing CAD, a user arbitrarily cut an area where it was determined that an intersection would occur between the restoration and an opposing tooth or between the restoration and an adjacent tooth. However, it is not easy for a user to manually determine an intersection area and the accuracy of intersection removal may vary according to the user's skill level.

### Disclosure

### Technical Problem

In order to solve the above problems, disclosed embodiments provide an image processing method for automatically determining an intersection (or interference) occurring between a designed restoration and an opposing tooth or between the restoration and an adjacent tooth and cutting (or removing) the intersection from the restoration and an apparatus for performing the image processing method.

### Technical Solution

An image processing method according to an embodiment includes obtaining an oral image including data about at least one tooth and data about a restoration, obtaining, based on the oral image, an intersection area (or interference area) where a first tooth of the at least one tooth and the restoration overlap each other, and obtaining a final image of the restoration by cutting (or removing) data corresponding to the intersection area from the data about the restoration.

The first tooth according to an embodiment may include at least one of an opposing tooth engaged with the restoration and an adjacent tooth adjacent to the restoration.

The obtaining of the intersection area according to an embodiment may include performing a ray-intersection test by generating virtual rays in a direction opposite to a normal direction of vertices included in data about the first tooth, and obtaining the intersection area based on the intersecting virtual rays.

The obtaining of the final image of the restoration according to an embodiment may include obtaining the final image of the restoration by moving the data corresponding to the intersection area by a certain distance and cutting the moved data corresponding to the intersection area from the data about the restoration.

The obtaining of the final image of the restoration according to an embodiment may include obtaining the final image of the restoration based on data about the first tooth corresponding to the intersection area.

The obtaining of the final image of the restoration according to an embodiment may include moving the data about the first tooth corresponding to the intersection area by a first distance, obtaining second data that connects the moved data to data about the first tooth which is not moved, and obtaining the final image of the restoration by cutting the second data from the data about the restoration.

The obtaining of the final image of the restoration according to an embodiment may include moving some data among the data about the first tooth corresponding to the intersection area or data of a larger area including the data about the first tooth corresponding to the intersection area by a first distance, obtaining second data that connects the moved data to the some data among data about the first tooth which is not moved or data of a larger area including the data about the first tooth which is not moved, and obtaining the final image of the restoration by cutting the second data from the data about the restoration.

The image processing method according to an embodiment may further include receiving a user input to set the first distance.

The image processing method according to an embodiment may further include displaying the oral image, wherein the displaying of the oral image includes displaying a degree of overlapping between the restoration and the first tooth in the intersection area in a color.

The image processing method according to an embodiment may further include displaying the final image of the restoration.

An image processing apparatus according to an embodiment includes a display, a memory storing one or more instructions, and a processor configured to execute the one or more instructions stored in the memory to obtain an oral image including data about at least one tooth and data about a restoration, obtain, based on the oral image, an intersection area where a first tooth of the at least one tooth and the restoration overlap each other, obtain a final image of the restoration by cutting data corresponding to the intersection area from the data about the restoration, and control the display to display the final image of the restoration.

### Advantageous Effects

An image processing apparatus and an image processing method according to disclosed embodiments may automatically determine an intersection area occurring between a designed restoration and an opposing tooth or between the restoration and an adjacent tooth and may automatically cut a portion of the restoration corresponding to the intersection area.

Accordingly, a user does not need to arbitrarily determine and cut the intersection area, and may easily obtain a final image of the restoration. Also, because the intersection area may be accurately cut, the quality of the restoration generated based on the final image of the restoration may be improved.

### Description of Drawings

The disclosure may be readily understood from the following detailed description in conjunction with the accompanying drawings, and reference numerals denote structural elements.
FIG. 1 is a view for describing an image processing system, according to a disclosed embodiment.
FIG. 2 is a flowchart illustrating an image processing method, according to an embodiment.
FIG. 3 is a view illustrating an example where an image processing apparatus displays an oral image, according to an embodiment.
FIG. 4 is a view referenced to describe a method in which an image processing apparatus obtains an intersection area, according to an embodiment.
FIG. 5 is a view illustrating screens that display an intersection area to be identified from other areas in an oral image, according to an embodiment.
FIGS. 6 to 8 are views for describing an operation in which an image processing apparatus obtains a final image of a restoration by cutting an intersection area from a restoration image, according to an embodiment.
FIG. 9 is a view illustrating a user interface for setting a distance between a restoration and an opposing tooth, according to an embodiment.
FIG. 10 is a view illustrating screens that display final images of a restoration, according to an embodiment.
FIG. 11 is a block diagram illustrating an image processing apparatus, according to an embodiment.

### Mode for Invention

Principles and embodiments of the disclosure will be described in detail in order to fully convey the scope of the disclosure and enable one of ordinary skill in the art to embody and practice the disclosure. The disclosed embodiments may be implemented in various forms.

The same reference numerals denote the same elements throughout the specification. Not all elements of the embodiments are described in the specification, and descriptions of matters well known in the art to which the disclosure pertains or repeated descriptions between the embodiments will not be given. The terms such as "part" and "portion" used herein denote those that may be implemented by software or hardware. According to embodiments, a plurality of parts or portions may be implemented by a single unit or element, or a single part or portion may include a plurality of units or elements. Hereinafter, principles and embodiments of the disclosure will be described in detail with the accompanying drawings.

It will be understood that when an element is referred to as being "connected to" another element, it may be "directly connected" to the other element, or "indirectly connected" to the other element, with intervening elements therebetween. It will be further understood that the terms "comprises," "comprising," "includes," and/or "including" used herein specify the presence of components, but do not preclude the presence or addition of one or more other components, unless otherwise specified.

It will be understood that, although the terms "first", "second", etc. may be used herein to describe various elements, these elements should not be limited by these terms. The above terms are used only to distinguish one component from another.

In the disclosure, an "object" may be a target to be imaged and may include a human, an animal, or a part of a human or animal. For example, an object may include a body part (e.g., an organ) or a phantom. Also, for example, an object may include a gypsum model that imitates an oral cavity, a denture such as an artificial tooth or a false tooth, or a teeth-shaped dentiform. For example, an object may include teeth, gums, at least a portion of an oral cavity, an artificial structure (e.g., an orthodontic appliance including brackets and a wire, an implant, an abutment, an artificial tooth, a dental restoration including an inlay and an onlay, or an orthodontic aid inserted into the oral cavity) that may be inserted into the oral cavity, and/or the teeth or the gums to which the artificial structure is attached.

The term "scanner" may refer to a device that obtains an image related to an object. The scanner may refer to a scanner that obtains an image related to an oral cavity used for oral treatment. For example, the scanner may be an intraoral scanner that may be inserted into an oral cavity. The intraoral scanner may be generally held and carried with one hand, and thus, may be referred to as a hand-held scanner. Alternatively, the scanner may be a table-top scanner that may be used for dental treatment. Also, the scanner may obtain at least one of a two-dimensional (2D) image and a three-dimensional (3D) image. Also, the scanner may obtain at least one 2D image of an oral cavity and generate a 3D image (or a 3D model) of the oral cavity based on the obtained at least one 2D image. Also, the scanner may obtain at least one 2D image of an oral cavity and transmit the at least one 2D image to an external device. The external device may generate a 3D image of the oral cavity based on the received at least one 2D image.

Also, "scanning oral cavity" may mean not only scanning an oral cavity itself but also scanning an artificial structure and/or another object representing or related to the oral cavity.

The term "image" may be a 2D image of an object or a 3D model or a 3D image three-dimensionally representing the object. For example, an image may be data required to two-dimensionally or three-dimensionally represent an object. For example, an image may refer to raw data or a raw image obtained from at least one camera. In detail, a raw image may be data obtained to generate an oral image required for diagnosis, and may be an image (e.g., a 2D frame image) obtained by at least one camera included in a scanner when the inside of a patient's oral cavity which is an object is scanned by using the scanner (e.g., intraoral scanner). Also, a raw image is an unprocessed and may refer to an original image obtained from a scanner.

A "3D oral model" may refer to a model that three-dimensionally models an oral cavity based on raw data obtained through a scanning operation of a scanner. Also, a "3D oral model" may refer to a structure that is three-dimensionally modeled based on data obtained when an object such as teeth, an impression body, or an artificial structure is scanned by a scanner. The 3D oral model is created by three-dimensionally modeling an internal structure of an oral cavity and may be referred to as a 3D scan model, a 3D model, or a tooth model. For example, a format of the 3D oral model may be one of, but not limited to, standard triangle language (STL), OBJ, and polygon file format (PLY). Also, a 3D oral model may include information such as geometric information, color, texture, and material about a 3D shape.

Also, a "polygon" may refer to a polygon that is a smallest unit used to represent a 3D shape of a 3D oral model. For example, a surface of a 3D oral model may be represented by triangular polygons. For example, a polygon may include at least three vertices and one face. The vertices may include information such as position, color, and normal line. A mesh may be an object in a 3D space formed by gathering a plurality of polygons. As the number of polygons representing a 3D oral model increases, an object may be represented in more detail.

Hereinafter, embodiments will be described in detail with reference to the drawings.

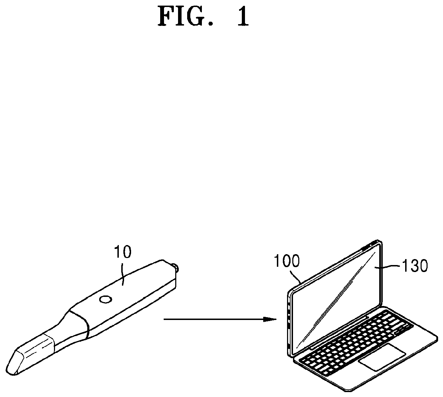

FIG. 1 is a view for describing an image processing system, according to a disclosed embodiment.

Referring to FIG. 1, an image processing system includes a scanner 10 and an image processing apparatus 100. The scanner 10 and the image processing apparatus 100 may communicate with each other through a communication network.

The scanner 10 is a device for scanning an object and may be used as a medical device for obtaining an image of the object. The scanner 10 may obtain an image of at least one of an oral cavity, an artificial structure, or a gypsum model that simulates the oral cavity or the artificial structure.

A scanner that may be inserted into the oral cavity such as the scanner 10 of FIG. 1 may be referred to as an intraoral scanner or a portable scanner. The scanner 10 may be a table-top scanner in addition to the hand-held scanner shown in FIG. 1.

The scanner 10 may obtain an image of an oral cavity including at least one tooth by being inserted into the oral cavity and scanning an object (e.g., an object or an impression body in the oral cavity such as teeth) in a non-contact manner. Also, the scanner 10 may scan the inside of a patient's oral cavity or an impression body that simulates the inside of the oral cavity by using at least one image sensor (e.g., an optical camera).

In order to image a surface of at least one of teeth and gums in the oral cavity and an artificial structure (e.g., an orthodontic appliance including brackets and a wire, an implant, an artificial tooth, or an orthodontic aid inserted into the oral cavity) that may be inserted into the oral cavity, the scanner 10 may obtain surface information of the object as raw data.

The raw data obtained by the scanner 10 may be at least one image obtained by at least one camera included in the scanner 10. In detail, the raw data may be at least one 2D frame image obtained when the scanner 10 performs a scanning operation. A 'frame image' may be referred to as a 'frame' or 'frame data'.

The scanner 10 may transmit the obtained raw data to the image processing apparatus 100 through a communication network. Alternatively, the scanner 10 may obtain a 3D model or a 3D image generated based on the raw data obtained by the at least one camera. The obtained 3D model or 3D image may be transmitted to the image processing apparatus 100.

Image data obtained by the scanner 10 may be transmitted to the image processing apparatus 100 connected through a wired or wireless communication network.

The image processing apparatus 100 may be any electronic device that is connected to the scanner through a wired or wireless communication network, and may receive data obtained by scanning an object from the scanner 10 and generate, process, display, and/or transmit an image based on the received data.

The image processing apparatus 100 may be a computing device such as, but not limited to, a smartphone, a laptop computer, a desktop computer, a PDA, or a tablet PC. Also, the image processing apparatus 100 may be provided as a server (or a server device) for processing an oral image.

In detail, the image processing apparatus 100 may generate at least one of information required for oral diagnosis, an image indicating an oral cavity, and a model used for oral treatment (e.g., a 3D model for teeth or a 3D model for generating a crown) based on data received from the scanner 10, and may display the generated information and image through a display 130.

For example, the scanner 10 may transmit raw data obtained through scanning to the image processing apparatus 100. In this case, the image processing apparatus 100 may generate a 3D oral image (3D oral model) that three-dimensionally represents an oral cavity based on the received raw data. The image processing apparatus 100 according to an embodiment may generate 3D data (e.g., surface data or mesh data) that three-dimensionally represents a shape of a surface of an object based on the received raw data. In this case, the 3D data may include a plurality of polygons.

Also, the image processing apparatus 100 may analyze, process, display, and/or transmit the generated image to an external device.

In another example, the scanner 10 may obtain raw data by scanning an object, may process the obtained raw data to generate an image corresponding to the object, and may transmit the image to the image processing apparatus 100. In this case, the image processing apparatus 100 may analyze, process, display, and/or transmit the received image.

In a disclosed embodiment, the image processing apparatus 100 is an electronic device capable of generating and displaying an image that three-dimensionally represents an object, which will be described below in detail.

When the image processing apparatus 100 according to an embodiment receives raw data obtained by scanning an object from the scanner 10, the image processing apparatus 100 may process the received raw data and generate a 3D image (or a 3D model). For convenience of explanation, a 3D image of an object generated by the image processing apparatus 100 will be referred to as 'scan data'.

For example, the scanner 10 may scan an oral cavity including at least one tooth. The image processing apparatus 100 according to an embodiment may receive raw data obtained by scanning the oral cavity from the scanner 10, may generate 3D scan data about the oral cavity including the at least one tooth based on the received raw data, and may display the generated 3D scan data (3D image) on the display 130.

The image processing apparatus 100 according to an embodiment may automatically determine an intersection occurring between a designed restoration and an opposing tooth or between the restoration and an adjacent tooth based on scan data about an oral cavity, and may obtain a final image of the restoration by cutting the intersection from the restoration.

Hereinafter, a method in which the image processing apparatus 100 according to an embodiment obtains a final image of a restoration by automatically cutting an intersection occurring between the designed restoration and an opposing tooth or between the restoration and an adjacent tooth will be described with reference to the drawings.

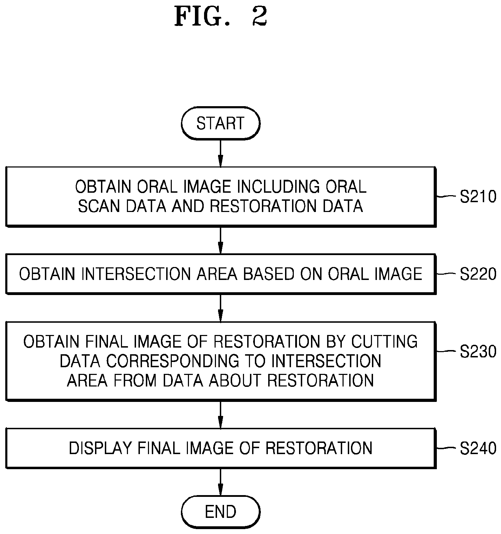

FIG. 2 is a flowchart illustrating an image processing method, according to an embodiment.

Referring to FIG. 2, the image processing apparatus 100 according to an embodiment may obtain an oral image including oral scan data and data about a restoration (S210).

For example, the image processing apparatus 100 may receive raw data obtained by scanning an oral cavity including at least one tooth from the scanner 10. The image processing apparatus 100 may obtain a 3D image or a 3D model of the oral cavity based on the received raw data. In this case, the 3D image of the oral cavity may include a plurality of objects in the oral cavity and 3D shape information of surfaces of the objects. For example, the 3D image of the oral cavity may be represented by a plurality of polygons, and a shape of each polygon may be a triangular shape. However, the disclosure is not limited thereto.

Alternatively, the image processing apparatus 100 may receive a 3D image of the oral cavity from the scanner 10 or an external device.

Also, the image processing apparatus 100 may identify a tooth needing treatment (target tooth) in the 3D image of the oral cavity and may design a restoration of the target tooth. For example, the image processing apparatus 100 may design a restoration of the target tooth based on 3D shape information of the target tooth. The image processing apparatus 100 may design a restoration of the target tooth by using a computer-aided design (CAD)/computer-aided manufacturing (CAM) program. However, the disclosure is not limited thereto.

Alternatively, the image processing apparatus 100 may transmit the 3D image of the oral cavity or data about the target tooth to the external device. The external device may design a restoration of the target tooth based on the 3D image or the data received from the image processing apparatus 100, and may transmit an image or data about the restoration to the image processing apparatus 100.

The image processing apparatus 100 according to an embodiment may obtain an oral image including 3D scan data obtained by scanning the oral cavity and data (hereinafter, 'restoration data') about the restoration. For example, the image processing apparatus 100 may obtain an oral image in which the restoration is attached to the target tooth by applying the restoration data to the 3D scan data about the oral cavity. In this case, the 3D scan data may include data about an upper jaw and data about a lower jaw, and tooth data included in the 3D scan data may be aligned data. For example, the image processing apparatus 100 may align the tooth data included in the 3D scan data by using pre-stored tooth model data. In this case, the image processing apparatus 100 may align the tooth data with the tooth model data, based on arch shape information, teeth shape information, and teeth size information included in the tooth data and the tooth model data. When the image processing apparatus 100 aligns the tooth data with the tooth model data, the image processing apparatus 100 may use any of various alignment algorithms such as iterative closest point (ICP). However, the disclosure is not limited thereto.

The image processing apparatus 100 according to an embodiment may obtain an intersection area based on the oral image (S220).

The image processing apparatus 100 may determine whether there is an area where an opposing tooth engaged with the restoration or an adjacent tooth adjacent to the restoration overlaps the restoration in the oral image. The image processing apparatus 100 may obtain an intersection area where the restoration overlaps the opposing tooth or the restoration overlaps the adjacent tooth by performing a ray-intersection test.

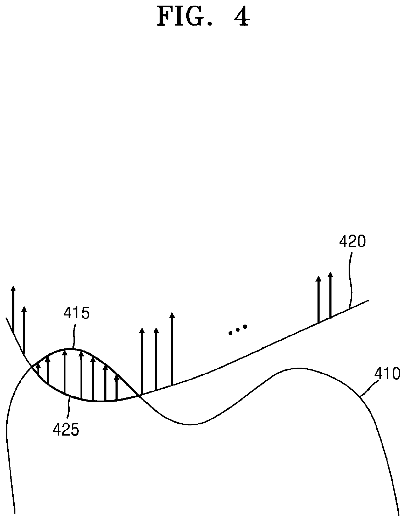

The ray-intersection test refers to generating a virtual ray in a direction opposite to a normal direction of a plurality of vertices included in data about the opposing tooth or the adjacent tooth and checking whether the generated virtual ray intersects the restoration data. The image processing apparatus 100 may obtain an overlapping intersection area based on a result of the ray-intersection test, which will be described in detail with reference to FIG. 4.

The image processing apparatus 100 according to an embodiment may obtain a final image of the restoration by cutting data corresponding to the intersection area from the restoration data (S230).

For example, when the virtual ray intersects the restoration data through the ray-intersection test in operation S220, the image processing apparatus 100 may cut data about the overlapping restoration. Also, the image processing apparatus 100 may obtain restoration data corresponding to a cut area, based on vertices of the opposing tooth or the adjacent tooth corresponding to intersecting vertices.

In detail, when the image processing apparatus 100 finds vertices or meshes of the restoration data intersecting the ray through the ray-intersection test, the image processing apparatus 100 may obtain a first area including vertices of the opposing tooth or the adjacent tooth in which the ray is generated.

The image processing apparatus 100 may move the vertices included in the first area by a first distance in an average normal direction of the vertices and may obtain first data including the moved vertices. In this case, the first distance may be set based on a user input.

The image processing apparatus 100 may obtain second data including the first data and data of a second area remaining after excluding the first area from the opposing tooth or adjacent tooth data. In this case, the second data may further include data connecting between the first data and a boundary of data of the second area.

The image processing apparatus 100 may obtain a final image of the restoration by subtracting the second data from the restoration data by using a Boolean method.

The image processing apparatus 100 according to an embodiment may display the final image of the restoration (S240).

The final image of the restoration may be an image having a shape in which an area engaged with the opposing tooth or the adjacent tooth is recessed.

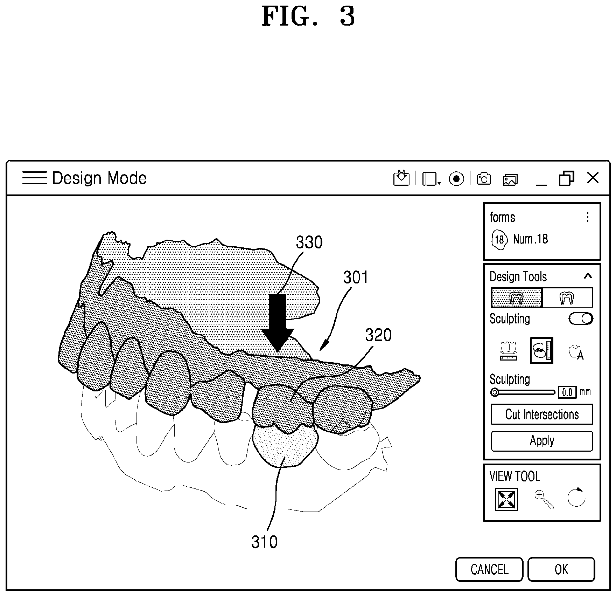

FIG. 3 is a view illustrating an example where an image processing apparatus displays an oral image, according to an embodiment.

Referring to FIG. 3, the image processing apparatus 100 according to an embodiment may display an oral image 301 including a designed restoration image (restoration data) along with a 3D image (3D scan data) of an oral cavity.

The image processing apparatus 100 may identify a tooth needing treatment (target tooth) in the 3D image of the oral cavity, and may design a restoration 310 of the target tooth. For example, the image processing apparatus 100 may design the restoration 310 of the target tooth by using a computer-aided design (CAD)/computer-aided manufacturing (CAM) program.

Alternatively, the image processing apparatus 100 may transmit the 3D image of the oral cavity or data about the target tooth to an external device, and the external device may design a restoration of the target tooth based on the received information. The external device may transmit data about the restoration to the image processing apparatus 100.

Accordingly, the image processing apparatus 100 may obtain data about the restoration 310, and may obtain the oral image 301 by applying the restoration data to the 3D image of the oral cavity. In this case, an intersection area where the restoration 310 and an opposing tooth or an adjacent tooth overlap each other may occur.

The image processing apparatus 100 according to an embodiment may obtain an overlapping intersection area between the restoration 310 and the opposing tooth or between the restoration 310 and the adjacent tooth. A method in which the image processing apparatus 100 according to an embodiment obtains an intersection area will be described with reference to FIG. 4.

FIG. 4 is a view referenced to describe a method in which an image processing apparatus obtains an intersection area, according to an embodiment.

In FIG. 4, for convenience of explanation, an intersection area occurring between a restoration and an opposing tooth will be described as an example.

Referring to FIG. 4, the image processing apparatus 100 according to an embodiment may obtain an intersection area where a restoration and an opposing tooth overlap each other by performing a ray-intersection test. The ray-intersection test may be performed based on restoration data 410 and opposing tooth data 420. The restoration data 410 may include surface data or mesh data representing a shape of a surface of the restoration 310, and may include a plurality of polygons. Also, the opposing tooth data 420 may include surface data or mesh data representing a shape of a surface of an opposing tooth 320, and may include a plurality of polygons.

For example, the image processing apparatus 100 may generate a virtual ray in a direction opposite to a normal direction of a plurality of vertices included in the opposing tooth data 420, and may check whether the generated virtual ray intersects the restoration data 410. When the virtual ray intersects the restoration data 410, the image processing apparatus 100 may obtain a corresponding area as an intersection area. For example, vertices or meshes of the restoration data 410 intersecting the virtual ray may be determined as restoration data 415 corresponding to the intersection area. Also, when the virtual ray intersects the restoration data 410, vertices generating the corresponding ray may be determined as opposing tooth data 425 corresponding to the intersection area.

The image processing apparatus 100 according to an embodiment may cut the intersection area from the restoration data 410 by using the opposing tooth data 425 corresponding to the intersection area. The image processing apparatus 100 needs to generate a virtual ray from the opposing tooth data 420 rather than the restoration data 410 in order to obtain the opposing tooth data 425 corresponding to the intersection area. Because vertices included in the restoration data 410 and vertices included in the opposing tooth data 420 are different from each other in number and position, when a virtual ray is generated from the restoration data 410 and vertices of the opposing tooth data 420 intersecting the virtual ray are found, only vertices corresponding to vertices of the restoration data are found. Accordingly, all vertices of the opposing tooth data 420 corresponding to the intersection area may not be found. Accordingly, in order to find all vertices included in the opposing tooth data 425 corresponding to the intersection area, the image processing apparatus 100 according to an embodiment may perform a ray-intersection test by generating virtual rays from vertices of the opposing tooth data 420.

Also, in order to cut the intersection area from the restoration data 410 by using a Boolean method, the opposing tooth data 425 corresponding to the intersection area is required. Accordingly, the image processing apparatus 100 according to an embodiment needs to perform a ray-intersection test by generating virtual rays from vertices of the opposing tooth data 420 rather than the restoration data 410.

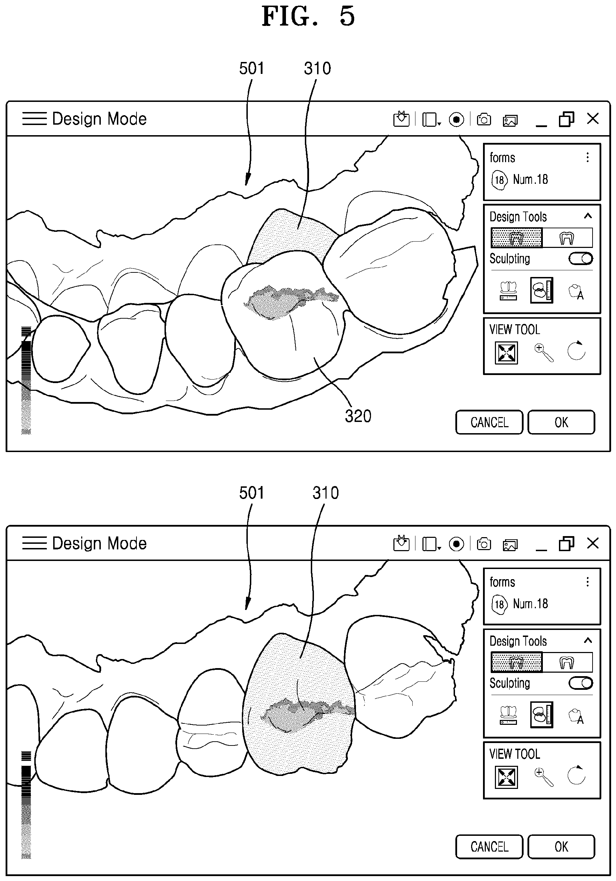

The image processing apparatus 100 according to an embodiment may display the obtained intersection area to be identified from other areas. For example, the image processing apparatus 100 may display the restoration data corresponding to the intersection area in a color determined according to a degree of overlapping, which will be described with reference to FIG. 5.

FIG. 5 is a view illustrating screens that display an intersection area to be identified from other areas in an oral image, according to an embodiment.

FIG. 5 is a view illustrating images that display an oral image of FIG. 3 from different viewpoints.

For example, oral images 501 and 502 of FIG. 5 are images displayed when the oral image 301 of FIG. 3 is viewed in a first direction 330. The oral image 501 of FIG. 5 is an oral image displaying both oral data about an upper jaw and oral data about a lower jaw, and the oral image 502 of FIG. 5 is an oral image displaying the oral data about the lower jaw including the restoration 310 and not displaying the oral data about the upper jaw. The image processing apparatus 100 may display or may not display the oral data about the upper jaw or the oral data about the lower jaw, based on a user input.

The first oral image 501 and the second oral image 502 may display an intersection area where the restoration 310 and the opposing tooth 320 overlap each other to be identified from other areas. For example, the first oral image 501 and the second oral image 502 may display an intersection area in a color that varies according to a degree of overlapping between the restoration 310 and the opposing tooth 320. In this case, a degree of overlapping may be displayed on the restoration 310 and may be determined through a ray-intersection test. For example, the image processing apparatus 100 may generate a virtual ray in a direction opposite to a normal direction of vertices of restoration data, and may check whether the generated virtual ray intersects opposing tooth data. When the virtual ray intersects the opposing tooth data, the image processing apparatus 100 may determine a degree of overlapping based on a distance from a starting point of the ray to a vertex where the ray intersects. The image processing apparatus 100 may determine that a degree of overlapping increases as a distance from a starting point of the ray to a vertex where the ray intersects increases, and may display a vertex or a mesh including the vertex in the restoration data from which the ray is generated in a color closer to a first color (e.g., red) as a degree of overlapping increases. Also, the image processing apparatus 100 may determine that a degree of overlapping decreases as a distance from a starting point of the ray to a vertex where the ray intersects decreases, and may display a vertex or a mesh including the vertex in the restoration data in which the ray is generated in a color closer to a second color (e.g., green) as a degree of overlapping decreases. However, the disclosure is not limited thereto.

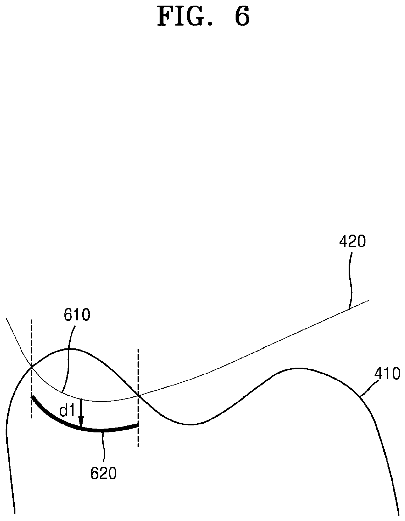

FIGS. 6 to 8 are views for describing an operation in which an image processing apparatus obtains a final image of a restoration by cutting an intersection area from a restoration image, according to an embodiment.

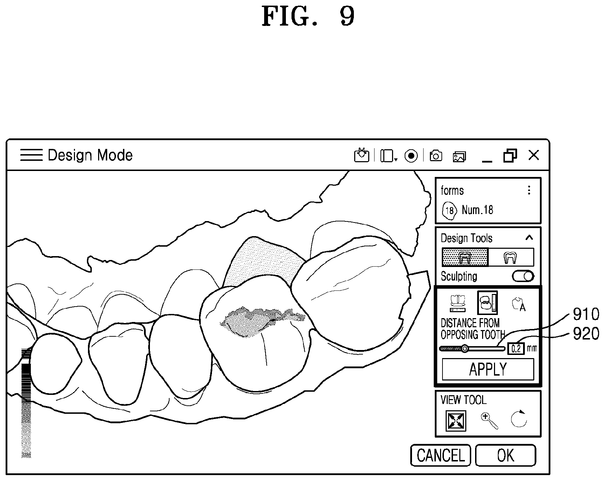

Referring to FIG. 6, the image processing apparatus 100 according to an embodiment may move the opposing tooth data 420 corresponding to an intersection area by a first distance d1. For example, the image processing apparatus 100 may move vertices or meshes included in opposing tooth data 610 (first data) corresponding to an intersection area by the first distance 1d in a normal direction of the vertices or the meshes and may obtain second data 620 including the moved vertices. In this case, the first distance d1 may be a value preset in the image processing apparatus 100, or may be set based on a user input through a user interface, which will be described in detail with reference to FIG. 9.

Alternatively, the image processing apparatus 100 according to an embodiment may move some data among the opposing tooth data 610 corresponding to the intersection area by the first distance d1 and may obtain the second data 620. For example, the image processing apparatus 100 may move data remaining after excluding data of an edge area of the opposing tooth data 610 by the first distance d1.

Alternatively, the image processing apparatus 100 according to an embodiment may move data of a larger area including the opposing tooth data 610 corresponding to the intersection area by the first distance d1 and may obtain the second data 620. For example, the image processing apparatus 100 may move data further including data adjacent to the edge area of the opposing tooth data 610 by the first distance d1.

When data is moved by the first distance d1, it may mean that vertices or meshes included in the data are moved by the first distance d1 in a normal direction of the vertices or the meshes.

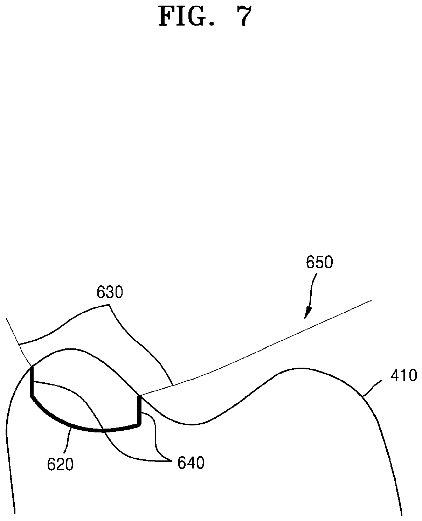

Referring to FIG. 7, the image processing apparatus 100 may obtain fourth data 640 that connects between a boundary of the second data 620 and data 630 (third data) remaining after excluding the first data 610 from the opposing tooth data 420. Accordingly, the image processing apparatus 100 may obtain fifth data 650 including the second data 620, the third data 630, and the fourth data 640.

Alternatively, the image processing apparatus 100 according to an embodiment may obtain the fourth data 640 that connects between some data among the third data 630 and a boundary of the second data 620 that is moved. For example, the fourth data 640 may be data that connects between a boundary of the second data 620 and data remaining after excluding data of an edge area of the third data 630. Accordingly, the image processing apparatus 100 may obtain the fifth data 650 including the second data 620, the some data of the third data 630, and the fourth data 640.

Alternatively, the image processing apparatus 100 may obtain the fourth data 640 that connects between data of a larger area including the third data 630 and a boundary of the second data 620 that is moved. For example, the fourth data 640 may be data that connects between a boundary of the second data 620 and data further including data adjacent to the edge area of the third data 630. Accordingly, the image processing apparatus 100 may obtain the fifth data 650 including the second data 620, the data of the larger area including the third data 630, and the fourth data 640.

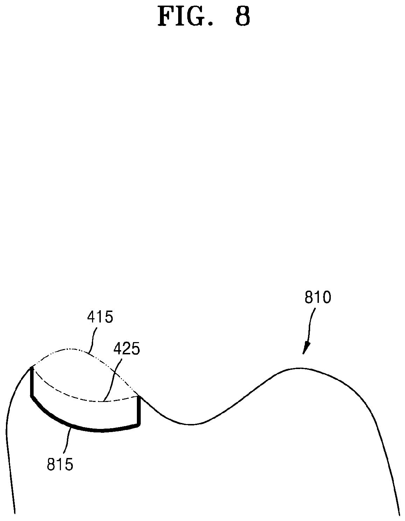

Referring to FIG. 8, the image processing apparatus 100 may obtain a final image 810 of the restoration by subtracting the fifth data 650 from the restoration data 410.

For example, the image processing apparatus 100 may obtain the final image 810 from which the restoration data 415 corresponding to the intersection area is removed, by cutting the fifth data 650 from the restoration data 410 by using a Boolean method. Also, the image processing apparatus 100 may obtain data 815 corresponding to an area where the restoration data is removed, based on the opposing tooth data 425 corresponding to the intersection area.

FIG. 9 is a view illustrating a user interface for setting a distance between a restoration and an opposing tooth, according to an embodiment.

Referring to FIG. 9, the image processing apparatus 100 according to an embodiment may display a slider 910 for adjusting a distance between a restoration and an opposing tooth on a user interface screen. The distance between the restoration and the opposing tooth may refer to the first distance d1 by which vertices included in the opposing tooth data 420 corresponding to an intersection area described with reference to FIG. 6 is moved.

Setting the distance between the restoration and the opposing tooth to a value other than 0 is to provide an offset. When the restoration is manufactured, an error may occur according to a device for manufacturing the restoration (e.g., a milling machine or a printer), and an offset distance may be set in order to correct the error.

The image processing apparatus 100 may receive a user input that adjusts the distance between the restoration and the opposing tooth by using the slider 910. For example, when the image processing apparatus 100 receives a user input that moves the slider 910 rightward, the image processing apparatus 100 may increase the first distance d1, and when the image processing apparatuses 100 receives a user input that moves the slider 910 leftward, the image processing apparatus 100 may reduce the first distance d1. Also, the image processing apparatus 100 may display the first distance d1 set by the slider 910 as a numerical value 920. However, the disclosure is not limited thereto.

When the distance between the restoration and the opposing tooth is set, the image processing apparatus 100 may obtain a final image of the restoration based on the set distance, which has been described in detail with reference to FIG. 6, and thus, the same description will be omitted.

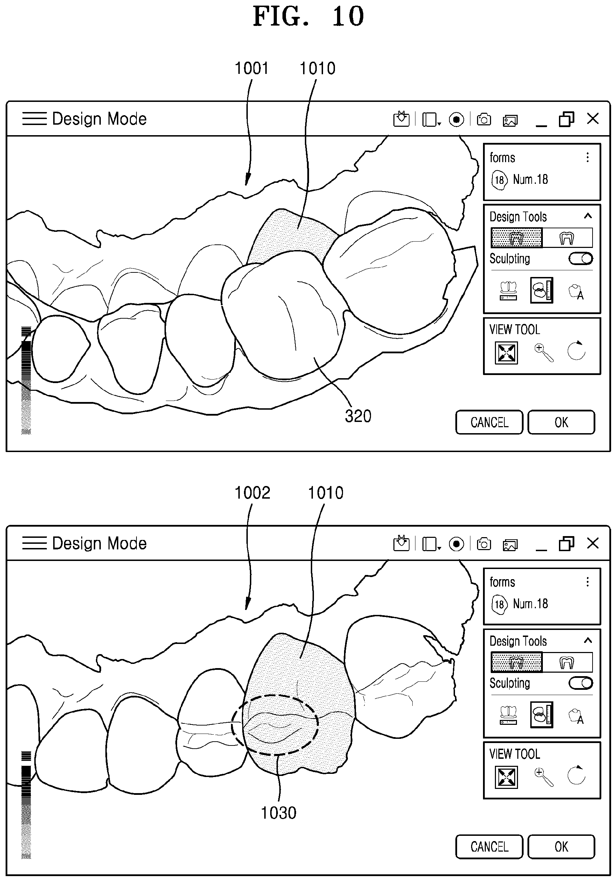

FIG. 10 is a view illustrating screens that display final images of a restoration, according to an embodiment.

Referring to FIG. 10, the image processing apparatus 100 according to an embodiment may display a final image of a restoration on a screen.

A first oral image 1001 of FIG. 10 is an oral image displaying oral data about an upper jaw and a lower jaw and a final image 1010 of a restoration, and a second oral image 1002 of FIG. 10 is an oral image displaying oral data about a lower jaw including the restoration and the final image 1010 of the restoration.

The image processing apparatus 100 may display or may not display the oral data about the upper jaw or the oral data about the lower jaw, based on a user input. Also, the image processing apparatus 100 may display only the final image 1010 of the restoration, without displaying the oral data about the upper jaw and the lower jaw. However, the disclosure is not limited thereto.

For example, the final image 1010 of the restoration displayed in the first oral image 1001 and the second oral image 1002 may include a recessed shape 1030 by removing an intersection area overlapping an opposing tooth. Also, compared to the first oral image 501 and the second oral image 502 of FIG. 5, the first oral image 1001 and the second oral image 1002 of FIG. 10 do not include an intersection area whose color varies according to a degree of overlapping.

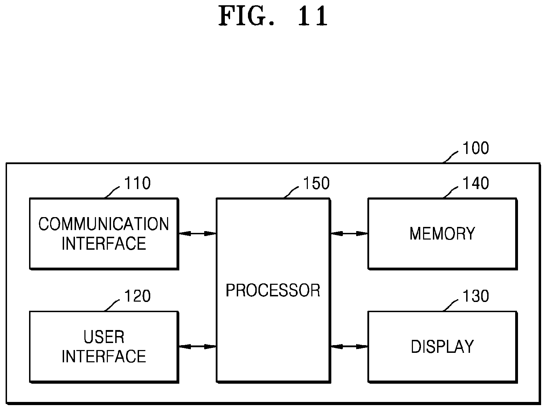

FIG. 11 is a block diagram illustrating an image processing apparatus, according to an embodiment.

An image processing method shown in FIG. 2 may be performed by the image processing apparatus 100. Accordingly, the image processing method of FIG. 2 may be a flowchart illustrating operations of the image processing apparatus 100.

Referring to FIG. 11, the image processing apparatus 100 may include a communication interface 110, a user interface 120, a display 130, a memory 140, and a processor 150.

The communication interface 110 may communicate with at least one external electronic device (e.g., the scanner 10, a server, or an external medical device) through a wired or wireless communication network. The communication interface 110 may communicate with the at least one external electronic device under the control of the processor 150.

In detail, the communication interface 110 may include at least one short-range communication module performing communication according to a communication standard such as Bluetooth, Wi-Fi, Bluetooth low energy (BLE), NFC/RFID, Wi-Fi Direct, UWB, or ZIGBEE.

Also, the communication interface 110 may further include a long-range communication module for performing communication with the server to support long-range communication according to a long-range communication standard. In detail, the communication interface 110 may include a long-range communication module that performs communication through a network for Internet communication. Also, the communication interface 110 may include a long-range communication module that performs communication through a communication network according to a communication standard such as 3G, 4G, and/or 5G.

Also, the communication interface 110 may include at least one port to be connected to the external electronic device through a wired cable, in order to communicate with the external electronic device (e.g., an intraoral scanner) by wire. Accordingly, the communication interface 110 may communicate with the external electronic device connected by wire through the at least one port.

The user interface 120 may receive a user input for controlling the image processing apparatus 100. The user interface 120 may include, but is not limited to, a touch panel for sensing a user's touch, a button for receiving the user's push operation, and a user input device including a mouse or keyboard for indicating or selecting one point on a user interface screen.

Also, the user interface 120 may include a voice recognition device for voice recognition. For example, the voice recognition device may be a microphone, and the voice recognition device may receive the user's voice command or voice request. Accordingly, the processor 150 may control an operation corresponding to the voice command or the voice request to be performed.

The display 130 may display a screen. In detail, the display 130 may display a certain screen under the control of the processor 150. In detail, the display 130 may display a user interface screen including an oral image generated based on data obtained when the scanner 10 scans a patient's oral cavity. Alternatively, the display 130 may display a user interface screen including an image of an object generated based on data obtained from the scanner 10.

Alternatively, the display 130 may display a user interface screen including information related to dental treatment of the patient.

The memory 140 may store at least one instruction. Also, the memory 140 may store at least one instruction executed by the processor 150. Also, the memory 140 may store at least one program executed by the processor 150. Also, the memory 140 may store data received from the scanner 10 (e.g., raw data obtained through scanning). Alternatively, the memory 140 may store an image that three-dimensionally represents an object. The memory 140 according to an embodiment may include one or more instructions for cutting some data from a restoration so as not to cause an intersection area between the restoration and an opposing tooth or between the restoration and an adjacent tooth. The memory 140 according to an embodiment may include one or more instructions for performing a method of the present disclosure to obtain an intersection area between a restoration and an opposing tooth or between the restoration and an adjacent tooth and obtain a final image of the restoration based on the intersection area.

The processor 150 controls an intended operation to be performed, by executing the one or more instructions stored in the memory 140. The at least one instruction may be stored in an internal memory included in the processor 150 or may be stored in the memory 140 included in a separate data processing device.

In detail, the processor 150 may control at least one element included in a data processing apparatus to perform an intended operation, by executing the at least one instruction. Accordingly, even when it is described that the processor performs certain operations, it may mean that the processor controls at least one element included in the data processing apparatus to perform certain operations.

The processor 150 according to an embodiment may generate scan data, based on raw data received from a 3D scanner, by executing one or more instructions stored in the memory 140. In this case, the raw data may include raw data obtained when an oral cavity including at least one tooth is scanned by the 3D scanner.

The processor 150 may obtain a 3D image or a 3D model of the oral cavity, based on the received raw data, by executing the one or more instructions stored in the memory 140.

The processor 150 may obtain an image or data about a restoration of a tooth needing treatment (target tooth) in the 3D image of the oral cavity, by executing the one or more instructions stored in the memory 140. For example, the processor 150 may identify the target tooth in the 3D image of the oral cavity and may design a restoration of the target tooth. The processor 150 may design a restoration of the target tooth, based on 3D shape information about the target tooth. The processor 150 may design a restoration of the target tooth, by using a computer-aided design (CAD)/computer-aided manufacturing (CAM) program. However, the disclosure is not limited thereto.

Alternatively, the processor 150 may transmit the 3D image of the oral cavity or data about the target tooth to an external device, and may receive an image or data about the restoration from the external device.

The processor 150 according to an embodiment may obtain an oral image including 3D scan data obtained by scanning the oral cavity and data about the restoration (hereinafter, 'restoration data'), by executing the one or more instructions stored in the memory 140. For example, the processor 150 may obtain an oral image in which the restoration is attached to the target tooth, by applying the restoration data to the 3D scan data about the oral cavity. In this case, the 3D scan data may include data about an upper jaw and data about a lower jaw, and tooth data included in the 3D scan data may be aligned data.

The processor 150 according to an embodiment may obtain an intersection area, based on the oral image, by executing the one or more instructions stored in the memory 140. The processor 150 may determine whether there is an area where an opposing tooth engaged with the restoration or an adjacent tooth adjacent to the restoration overlaps the restoration in the oral image. The processor 150 may obtain an intersection area where the restoration and the opposing tooth or the adjacent tooth overlap each other, by performing a ray-intersection test. The ray-intersection test refers to generating a virtual ray in a direction opposite to a normal direction of a plurality of vertices included in data about the opposing tooth or the adjacent tooth and checking whether the generated virtual ray intersects the restoration data.

The processor 150 according to an embodiment may obtain a final image of the restoration, by cutting data corresponding to the intersection area from the restoration data, by executing the one or more instructions stored in the memory 140. For example, when the virtual ray intersects the restoration data through the ray-intersection test, the processor 150 may cut data about the intersecting restoration. Also, the processor 150 may obtain restoration data corresponding to a cut area, based on vertices of the opposing tooth or the adjacent tooth corresponding to intersecting vertices.

In detail, when the processor 150 finds vertices of the restoration data intersecting the ray through the ray-intersection test, the processor 150 may obtain a first area including vertices of the opposing tooth or the adjacent tooth in which the ray is generated.

The processor 150 may move the vertices included in the first area by a first distance in a normal direction of the vertices and may obtain first data including the moved vertices. In this case, the first distance may be set based on a user input.

The processor 150 may obtain second data including the first data and data of a second area remaining after excluding the first area from opposing tooth or adjacent tooth data. In this case, the second data may further include data connecting between the first data and a boundary of data of the second area.

The processor 150 may obtain a final image of the restoration by subtracting the second data from the restoration data by using a Boolean method.

The processor 150 according to an embodiment may display the final image of the restoration.

The processor 150 according to an embodiment may include at least one internal processor and a memory device (e.g., a RAM or a ROM) for storing at east one of programs, instructions, signals, and data to be processed or used by the internal processor.

Also, the processor 150 may include a graphics processing unit for graphics processing corresponding to video. Also, the processor may be implemented as a system-on-chip (SoC) in which a core and a GPU are integrated. Also, the processor may include a single core or a multi-core. For example, the processor may include a dual core, a triple core, a quad core, a hexa core, an octa core, a deca core, a dodeca core, or a hexadecimal core.

In a disclosed embodiment, the processor 150 may generate an image based on a 2D image received from the scanner 10.

In detail, the communication interface 110 may receive data obtained by the scanner 10, for example, raw data obtained through scanning, under the control of the processor 150. The processor 150 may generate a 3D image that three-dimensionally represents an object based on the raw data received by the communication interface 110. For example, the scanner 10 may include a left (L) camera corresponding to a left field of view and a right (R) camera corresponding to a right field of view, in order to restore a 3D image according to an optical triangulation method. A 3D scanner may obtain L image data corresponding to the left field of view and R image data corresponding to the right field of view from the L camera and the R camera. Subsequently, the 3D scanner may transmit raw data including the L image data and the R image data to the communication interface 110 of the image processing apparatus 100.

Next, the communication interface 110 may transmit the received raw data to the processor 150, and the processor 150 may generate an image that three-dimensionally represents the object based on the received raw data.

Also, the processor 150 may control the communication interface 110 to directly receive an image that three-dimensionally represents the object from an external server or a medical device. In this case, the processor may obtain a 3D image, without generating a 3D image based on the raw data.

According to a disclosed embodiment, the processor 150 performing operations such as 'extracting', 'obtaining', and 'generating' may include not only the processor 150 directly performing the above operations by executing at least one instruction but also controlling other components to perform the above operations.

In order to implement disclosed embodiments of the disclosure, the image processing apparatus 100 may include only some of the components illustrated in FIG. 11 or may include more components than the components illustrated in FIG. 11.

Also, the image processing apparatus 100 may store and execute dedicated software lined to the scanner 10. The dedicated software may be referred to as a dedicated program, a dedicated tool, or a dedicated application. When the image processing apparatus 100 operates in conjunction with the scanner 10, the dedicated software stored in the image processing apparatus 100 may be connected to the scanner 10 and may receive data obtained by scanning an object in real time. For example, there is dedicated software for processing data obtained by scanning an object using i500 that is a 3D scanner of Medit. In detail, Medit produces and distributes `Medit Link' that is software for processing, managing, using, and/or transmitting data obtained by a 3D scanner (e.g., i500). Because the 'dedicated software' refers to a program, tool, or application that may operate in conjunction with a 3D scanner, various 3D scanners developed and sold by various manufacturers may be used in common. Also, the dedicated software may be produced and distributed separately from the 3D scanner that scans an object.

The image processing apparatus 100 may store and execute dedicated software corresponding to the i500 product. The dedicated software may perform at least one operation for obtaining, processing, storing, and/or transmitting an image. The dedicated software may be stored in the processor. Also, the dedicated software may provide a user interface for using data obtained by the 3D scanner. A user interface screen provided by the dedicated software may include an image generated according to a disclosed embodiment.

An image processing method according to an embodiment of the disclosure may be implemented as a program command executable by various computer means and may be recorded on a computer-readable medium. Also, an embodiment of the disclosure may be a computer-readable storage medium having recorded thereon at least one program including at least one instruction for executing an image processing method.

The computer-readable storage medium may include program commands, data files, data structures, and the like separately or in combinations. Examples of the computer-readable storage medium may include a magnetic medium such as a hard disk, a floppy disk, or a magnetic tape, an optical medium such as a compact disc read-only memory (CD-ROM) or a digital versatile disc (DVD), a magneto-optical medium such as a floptical disk, and a hardware device configured to store and execute program commands such as a ROM, a RAM, or a flash memory.

A machine-readable storage medium may be provided as a non-transitory storage medium. The term 'non-transitory storage medium' may refer to a tangible device. Also, the 'non-transitory storage medium' may include a buffer where data is temporarily stored.

According to an embodiment of the disclosure, image processing methods according to various embodiments of the disclosure may be provided in a computer program product. The computer program product may be distributed in the form of a machine-readable storage medium (e.g., a CD-ROM). Alternatively, the computer program product may be distributed (e.g., downloaded or uploaded) online via an application store (e.g., Play Store^{™}) or between two user devices (e.g., smartphones) directly. In detail, the computer program product according to a disclosed embodiment may include a storage medium having recorded thereon a program including at least one instruction to execute an image processing method according to a disclosed embodiment.

Although embodiments have been described in detail above, the scope of the disclosure is not limited thereto, and various modifications and improvements made by one of ordinary skill in the art by using the basic concept of the disclosure defined by the claims are also within the scope of the disclosure.

## Claims

1. An image processing method comprising:
obtaining an oral image comprising data about at least one tooth and data about a restoration;
obtaining, based on the oral image, an intersection area where a first tooth of the at least one tooth and the restoration overlap each other; and
obtaining a final image of the restoration by cutting data corresponding to the intersection area from the data about the restoration.

2. The image processing method of claim 1, wherein the first tooth comprises at least one of an opposing tooth engaged with the restoration and an adjacent tooth adjacent to the restoration.

3. The image processing method of claim 1, wherein the obtaining of the intersection area comprises:
performing a ray-intersection test by generating virtual rays in a direction opposite to a normal direction of vertices included in data about the first tooth; and
obtaining the intersection area based on the intersecting virtual rays.

4. The image processing method of claim 1, wherein the obtaining of the final image of the restoration comprises obtaining the final image of the restoration by moving the data corresponding to the intersection area by a certain distance and cutting the moved data corresponding to the intersection area from the data about the restoration.

5. The image processing method of claim 1, wherein the obtaining of the final image of the restoration comprises obtaining the final image of the restoration based on data about the first tooth corresponding to the intersection area.

6. The image processing method of claim 5, wherein the obtaining of the final image of the restoration comprises:
moving the data about the first tooth corresponding to the intersection area by a first distance;
obtaining second data that connects the moved data to data about the first tooth which is not moved; and
obtaining the final image of the restoration by cutting the second data from the data about the restoration.

7. The image processing method of claim 5, wherein the obtaining of the final image of the restoration comprises:
moving some data among the data about the first tooth corresponding to the intersection area or data of a larger area comprising the data about the first tooth corresponding to the intersection area by a first distance;
obtaining second data that connects the moved data to the some data among data about the first tooth which is not moved or data of a larger area comprising the data about the first tooth which is not moved; and
obtaining the final image of the restoration by cutting the second data from the data about the restoration.

8. The image processing method of claim 6, further comprising receiving a user input to set the first distance.

9. The image processing method of claim 1, further comprising displaying the oral image,
wherein the displaying of the oral image comprises displaying a degree of overlapping between the restoration and the first tooth in the intersection area in a color.

10. The image processing method of claim 1, further comprising displaying the final image of the restoration.

11. An image processing apparatus comprising:
a display;
a memory storing one or more instructions; and
a processor configured to execute the one or more instructions stored in the memory to
obtain an oral image comprising data about at least one tooth and data about a restoration,
obtain, based on the oral image, an intersection area where a first tooth of the at least one tooth and the restoration overlap each other,
obtain a final image of the restoration by cutting data corresponding to the intersection area from the data about the restoration, and
control the display to display the final image of the restoration.

12. The image processing apparatus of claim 11, wherein the first tooth comprises at least one of an opposing tooth engaged with the restoration and an adjacent tooth adjacent to the restoration.

13. The image processing apparatus of claim 11, wherein the processor is further configured to execute the one or more instructions stored in the memory to
perform a ray-intersection test by generating virtual rays in a direction opposite to a normal direction of vertices included in data about the first tooth, and
obtain the intersection area, based on the intersecting virtual rays.

14. The image processing apparatus of claim 11, wherein the processor is further configured to execute the one or more instructions stored in the memory to obtain the final image of the restoration by moving the data corresponding to the intersection area by a certain distance and cutting the moved data corresponding to the intersection area from the data about the restoration.

15. The image processing apparatus of claim 11, wherein the processor is further configured to obtain the final image of the restoration based on data about the first tooth corresponding to the intersection area.

16. The image processing apparatus of claim 15, wherein the processor is further configured to execute the one or more instructions stored in the memory to
move the data about the first tooth corresponding to the intersection area by a first distance,
obtain second data that connects the moved data to data about the first tooth which is not moved, and
obtain the final image of the restoration by cutting the second data from the data about the restoration.

17. The image processing apparatus of claim 15, wherein the processor is further configured to execute the one or more instructions stored in the memory to
move some data among the data about the first tooth corresponding to the intersection area or data of a larger area comprising the data about the first tooth corresponding to the intersection area by a first distance,
obtain second data that connects the moved data to some data among data about the first tooth which is not moved or data of a larger area comprising the data about the first tooth which is not moved, and
obtain the final image of the restoration by cutting the second data from the data about the restoration.

18. The image processing apparatus of claim 16, further comprising a user interface configured to receive a user input to set the first distance.

19. The image processing apparatus of claim 11, wherein the processor is further configured to execute the one or more instructions stored in the memory to control the display to display a degree of overlapping between the restoration and the first tooth in the intersection area in a color.

20. A computer-readable recording medium having recorded thereon a program comprising at least one instruction for executing an image processing method on a computer, wherein the image processing method comprises:
obtaining an oral image comprising data about at least one tooth and data about a restoration;
obtaining, based on the oral image, an intersection area where a first tooth of the at least one tooth and the restoration overlap each other; and
obtaining a final image of the restoration by cutting data corresponding to the intersection area from the data about the restoration.
